# EUROPEAN PATENT APPLICATION

(11) **EP 2 604 297 A1**
(43) Date of publication of application: **19.06.2013**
(21) Application number: 12196397.9
(22) Date of filing: 10.12.2012
(51) Int. Cl.: A61L 9/01, A61L 9/012, A61Q 15/00, C11D 3/00

(54) **Novel malodor counteractant**

(30) Priority: 12.12.2011 US 201113316705
(71) Applicant: International Flavors & Fragrances, Inc., New York, NY 10019 (US)
(72) Inventor: Monteleone, Michael G., Hazlet, NJ New Jersey 07730 (US); Tabert, Matthias H., Arverne, NY New York 11692 (US); Levorse, Jr., Anthony T., Westfield, NJ New Jersey 07090 (US)
(74) Representative: Lawrence, John

(57) **Abstract**

The present invention pertains to a method of counteracting a malodor by introducing a malodor counteracting effective amount of methyl 3-methyl cyclohexane carboxylate.

## Description

### Field of the Invention

The present invention relates to a novel malodor counteractant and method of use and composition thereof.

### Background of the Invention

There is a particular effort in the fragrance industry for novel compounds to treat and control malodors. "Malodor" is a term used to describe undesirable or unpleasant odor. Common sources of malodors include body perspiration, smoke, environmental odor such as mold and mildew, bathroom, and etc. Conventional perfumes including a variety of fragrance materials are developed to mask malodors, which generally function via two mechanisms: first, the fragrance materials blend with the malodor compound to provide a different and more desirable aroma; and second, the fragrance materials are employed to overwhelm the malodor compound. However, a large quantity of fragrance materials is required for both mechanisms, which in itself is often undesirable. Thus, there remains a need for new chemicals that are effective in counteracting malodors.

### Summary of the Invention

The present invention provides a new use of methyl 3-methyl cyclohexane carboxylate. Methyl 3-methyl cyclohexane carboxylate, previously used in fragrance, is now surprisingly found to have unexpected advantageous properties in counteracting malodors.

One embodiment of the invention relates to a method of counteracting a malodor in a substrate or air space comprising the step of introducing a malodor counteracting effective amount of methyl 3-methyl cyclohexane carboxylate.

Another embodiment of the invention relates to a malodor counteracting composition comprising methyl 3-methyl cyclohexane carboxylate.

These and other embodiments of the present invention will be apparent by reading the following specification.

### Detailed Description of the Invention

Methyl 3-methyl cyclohexane carboxylate can be prepared according to the procedure detailed in U.S. Patent No. 7,632,792. Methyl 3-methyl cyclohexane carboxylate is also commercially available at International Flavors & Fragrances Inc. ("IFF", New York, NY, USA).

Methyl 3-methyl cyclohexane carboxylate is surprisingly found to possess unexpected advantages in malodor counteracting applications such as body perspiration, environmental odor such as mold and mildew, bathroom, and etc. It substantially eliminates the perception of malodors and/or prevents the formation of such malodors, thus, can be utilized with a vast number of functional products.

Examples of the functional products are provided herein to illustrate the various aspects of the present invention. However, they do not intend to limit the scope of the present invention. The functional products may include, for example, a conventional room freshener (or deodorant) composition such as room freshener sprays, an aerosol or other spray, fragrance diffusers, a wick or other liquid system, or a solid, for instance candles or a wax base as in pomanders and plastics, powders as in sachets or dry sprays or gels, as in solid gel sticks, clothes deodorants as applied by washing machine applications such as in detergents, powders, liquids, whiteners or fabric softeners, fabric refreshers, linen sprays, closet blocks, closet aerosol sprays, or clothes storage areas or in dry cleaning to overcome residual solvent notes on clothes, bathroom accessories such as paper towels, bathroom tissues, sanitary napkins, towellets, disposable wash cloths, disposable diapers, and diaper pail deodorants, cleansers such as disinfectants and toilet bowl cleaners, cosmetic products such as antiperspirant and deodorants, general body deodorants in the form of powders, aerosols, liquids or solid, or hair care products such as hair sprays, conditioners, rinses, hair colors and dyes, permanent waves, depilatories, hair straighteners, hair groom applications such as pomade, creams and lotions, medicated hair care products containing such ingredients as selenium sulphide, coal tar or salicylates, or shampoos, or foot care products such as foot powders, liquids or colognes, after shaves and body lotions, or soaps and synthetic detergents such as bars, liquids, foams or powders, odor control such as during manufacturing processes, such as in the textile finishing industry and the printing industry (inks and paper), effluent control such as in processes involved in pulping, stock yard and meat processing, sewage treatment, garbage bags, or garbage disposal, or in product odor control as in textile finished goods, rubber finished goods or car fresheners, agricultural and pet care products such as dog and hen house effluents and domestic animal and pet care products such as deodorants, shampoo or cleaning agents, or animal litter material and in large scale closed air systems such as auditoria, and subways and transport systems.

Thus, it will be seen that the composition of the invention is usually one in which the malodor counteractant is present together with a carrier by means of which or from which the malodor counteractant can be introduced into air space wherein the malodor is present, or a substrate on which the malodor has deposited. For example, the carrier can be an aerosol propellant such as a chlorofluoro-methane, or a solid such as a wax, plastics material, rubber, inert powder or gel. In a wick-type air freshener, the carrier is a substantially odorless liquid of low volatility. In several applications, a composition of the invention contains a surface active agent or a disinfectant, while in others, the malodor counteractant is present on a fibrous substrate. In many compositions of the invention there is also present a fragrance component which imparts a fragrance to the composition. The fragrances stated above can all be employed.

Malodor counteracting effective amount is understood to mean the amount of the inventive malodor counteractant employed in a functional product that is organoleptically effective to abate a given malodor while reducing the combined intensity of the odor level, wherein the given malodor is present in air space or has deposited on a substrate. The exact amount of malodor counteractant agent employed may vary depending upon the type of malodor counteractant, the type of the carrier employed, and the level of malodor counteractancy desired. In general, the amount of malodor counteractant agent present is the ordinary dosage required to obtain the desired result. Such dosage is known to the skilled practitioner in the art. In a preferred embodiment, when used in conjunction with malodorous solid or liquid functional products, e.g., soap and detergent, methyl 3-methyl cyclohexane carboxylate may be present in an amount ranging from about 0.005 to about 50 weight percent, preferably from about 0.01 to about 20 weight percent, more preferably from about 0.05 to about 10 weight percent, and even more preferably from about 0.1 to about 5 weight percent. When used in conjunction with malodorous gaseous functional products, methyl 3-methyl cyclohexane carboxylate may be present in an amount ranging from about 0.2 milligrams (mg) to about 2 grams (g) per cubic meter of air, preferably from about 0.4 mg to about 0.8 g per cubic meter of air, more preferably from about 2 mg to about 0.4 g per cubic meter of air, and even more preferably from about 4 mg to about 0.2 g per cubic meter of air.

The following are provided as specific embodiments of the present invention. Other modifications of this invention will be readily apparent to those skilled in the art. Such modifications are understood to be within the scope of this invention. All reagents were purchased from Sigma-Aldrich, Inc. unless otherwise noted. Further, as used herein all percentages are weight percent unless otherwise noted, mL is understood to be milliliter, and oz is understood to be ounce. IFF as used in the examples is understood to mean International Flavors & Fragrances Inc., New York, NY, USA.

### EXAMPLE I

**Establishment of Malodor Models:** A number of malodorous chemicals have been identified, which are associated with different types of malodors. Commercial samples of these chemicals (commercially available at Sigma-Aldrich Inc.) were thus used in the present invention as malodorous model compounds to assess the effectiveness of methyl 3-methyl cyclohexane carboxylate in counteracting malodors.

A model of bathroom malodor was established using a solution of isovaleric acid ("IVA") in diethyl phthalate ("DEP") (10% by weight). A model of sweat malodor was established using a solution of 3-methyl-2-hexanoic acid in DEP (5% by weight). A model of mold/mildew malodors was established using a solution of 2-ethyl-1-hexanol in DEP (20% by weight). A model of smoke malodor was established by applying a solution of 3-acetyl pyridine in DEP (5% by weight) onto a piece of cloth.

**Preparation of Test Samples:** Methyl 3-methyl cyclohexane carboxylate was prepared at a series of concentrations, ranging from 0.1% to 5% in DEP, with corresponding vapor phase concentrations ranging from about 4 µg to about 0.2 mg per liter of air space.

A malodor material as established above (1 g) and a solution of methyl 3-methyl cyclohexane carboxylate in DEP (1 g) were pipetted into separate aluminum weighing dishes located at the bottom of a 32 oz. jar (1 L). For negative control samples (Malodor Alone), the malodor material (0.5 g) was pipetted into each of the two dishes located at the bottom of jar. Jars were then capped and the samples were allowed to equilibrate for 24 hours before testing.

**Testing Procedure:** Test samples were presented in a blind and pseudorandom order to a group of trained panelists (consisting of 12-17 females with a mean age of 40 years). The panelists were instructed to take the following steps: i) sniff malodor reference jars for familiarization prior to each testing session; ii) uncap a jar; iii) place their noses at a distance of about 3-4 inches above the opening; iv) take short sniffs for about 2 -3 seconds; and v) enter a rating of overall intensity and malodor intensity on a handheld computer.

The overall and malodor intensity was rated using the Labeled Magnitude Scale (LMS) [Green, et al., Chemical Senses, 21(3), Jun 1996, 323-334]. Mean ("Malodor Intensity") and standard error of the mean ("SE", ±) were obtained, where 0 represents "No Sensation" and 100 represents "The Strongest Imaginable Malodor Sensation". Percent malodor reduction ("%MOR") represents the perceived reduction in mean malodor intensity of the sample containing the malodor in the presence of methyl 3-methyl cyclohexane carboxylate relative to the negative control (Malodor Alone).

### EXAMPLE II

### Determination of the Bathroom Malodor Counteracting Effect of Methyl 3-Methyl Cyclohexane

The results for the bathroom malodor test were as follows:

| **No.** | **Group** | **Malodor Intensity** | **SE** | **%MOR** |
|---|---|---|---|---|
| 1 | Bathroom Malodor Alone | 20.08 | 1.04 | |
| | Methyl 3-Methyl Cyclohexane Carboxylate (0.1%, 4 µg/L) | 8.65 | 1.29 | 56.93 |
| 2 | Bathroom Malodor Alone | 16.23 | 1.06 | |
| | Methyl 3-Methyl Cyclohexane Carboxylate (0.5%, 21 µg/L) | 5.44 | 1.33 | 66.47 |
| 3 | Bathroom Malodor Alone | 19.36 | 1.03 | |
| | Methyl 3-Methyl Cyclohexane Carboxylate (1%, , 42 µg/L) | 2.69 | 1.27 | 86.11 |
| 4 | Bathroom Malodor Alone | 18.76 | 1.05 | |
| | Methyl 3-Methyl Cyclohexane Carboxylate (5%, 208 µg/L) | 1.71 | 1.31 | 90.86 |

The above test demonstrated that methyl 3-methyl cyclohexane carboxylate is effective in counteracting the bathroom malodor.

### EXAMPLE III

### Determination of the Sweat Malodor Counteracting Effect of Methyl 3-Methyl Cyclohexane Carboxylate

The results for the sweat malodor test were as follows:

| **No.** | **Group** | **Malodor Intensity** | **SE** | **%MOR** |
|---|---|---|---|---|
| 1 | Sweat Malodor Alone | 20.28 | 1.04 | |
| | Methyl 3-Methyl Cyclohexane Carboxylate (0.1%, 4 µg/L) | 4.48 | 1.32 | 77.92 |
| 2 | Sweat Malodor Alone | 18.69 | 1.05 | |
| | Methyl 3-Methyl Cyclohexane Carboxylate (0.5%, 21 µg/L) | 5.12 | 1.27 | 72.63 |
| 3 | Sweat Malodor Alone | 20.18 | 1.04 | |
| | Methyl 3-Methyl Cyclohexane Carboxylate (1%, , 42 µg/L) | 5.42 | 1.17 | 73.14 |
| 4 | Sweat Malodor Alone | 19.18 | 1.06 | |
| | Methyl 3-Methyl Cyclohexane Carboxylate (5%, 208 µg/L) | 3.55 | 1.3 | 81.47 |

The above test demonstrated that methyl 3-methyl cyclohexane carboxylate is effective in counteracting the sweat malodor.

### EXAMPLE IV

### Determination of the Mold/Mildew Malodor Counteracting Effect of Methyl 3-Methyl Cyclohexane Carboxylate

The results for the mold/mildew malodor test were as follows:

| **No.** | **Group** | **Malodor Intensity** | **SE** | **%MOR** |
|---|---|---|---|---|
| 1 | Mold/Mildew Malodor Alone | 16.47 | 1.12 | |
| | Methyl 3-Methyl Cyclohexane Carboxylate (0.1%, 4 µg/L) | 9.88 | 1.15 | 40.01 |
| 2 | Mold/Mildew Malodor Alone | 17.86 | 1.06 | |
| | Methyl 3-Methyl Cyclohexane Carboxylate (0.5%, 21 µg/L) | 5.44 | 1.24 | 69.56 |
| 3 | Mold/Mildew Malodor Alone | 18.92 | 1.04 | |
| | Methyl 3-Methyl Cyclohexane Carboxylate (1%, , 42 µg/L) | 4.44 | 1.26 | 76.51 |
| 4 | Mold/Mildew Malodor Alone | 19.74 | 1.04 | |
| | Methyl 3-Methyl Cyclohexane Carboxylate (5%, 208 µg/L) | 3.34 | 1.23 | 83.07 |

The above test demonstrated that methyl 3-methyl cyclohexane carboxylate is effective in counteracting the mold/mildew malodor.

### EXAMPLE V

### Determination of the Smoke Malodor Counteracting Effect of Methyl 3-Methyl Cyclohexane Carboxylate

The results for the smoke malodor test were as follows:

| **No.** | **Group** | **Malodor Intensity** | **SE** | **%MOR** |
|---|---|---|---|---|
| 1 | Smoke Malodor Alone | 20.86 | 1.06 | |
| | Methyl 3-Methyl Cyclohexane Carboxylate (0.1%, 4 µg/L) | 10.02 | 1.16 | 51.95 |
| 2 | Smoke Malodor Alone | 21.3 | 1.03 | |
| | Methyl 3-Methyl Cyclohexane Carboxylate (0.5%, 21 µg/L) | 7.23 | 1.34 | 66.04 |
| 3 | Smoke Malodor Alone | 21.33 | 1.04 | |
| | Methyl 3-Methyl Cyclohexane Carboxylate (1%, , 42 µg/L) | 4.78 | 1.24 | 77.57 |
| 4 | Smoke Malodor Alone | 21.22 | 1.03 | |
| | Methyl 3-Methyl Cyclohexane Carboxylate (5%, 208 µg/L) | 4.23 | 1.29 | 80.07 |

The above test demonstrated that methyl 3-methyl cyclohexane carboxylate is effective in counteracting the smoke malodor.

## Claims

1. A method of counteracting a malodor in air space or a substrate comprising the step of introducing a malodor counteracting effective amount of methyl 3-methyl cyclohexane carboxylate.

2. The method of claim 1, wherein the compound is used in a malodorous solid or liquid functional product, and wherein the malodor counteracting effective amount is from about 0.005% to about 50% by weight.

3. The method of claim 1, wherein the compound is used in a malodorous solid or liquid functional product, and wherein the malodor counteracting effective amount is from about 0.01% to about 20% by weight.

4. The method of claim 1, wherein the compound is used in a malodorous solid or liquid functional product, and wherein the malodor counteracting effective amount is from about 0.05% to about 10% by weight.

5. The method of claim 1, wherein the compound is used in a malodorous solid or liquid functional product, and wherein the malodor counteracting effective amount is from about 0.1% to about 5% by weight.

6. The method of claim 1, wherein the malodor in air space, and wherein the malodor counteracting effective amount is from about 0.2 mg to about 2 g per cubic meter of air.

7. The method of claim 1, wherein the malodor in air space, and wherein the malodor counteracting effective amount is from about 0.4 mg to about 0.8 g per cubic meter of air.

8. The method of claim 1, wherein the malodor in air space, and wherein the malodor counteracting effective amount is from about 2 mg to about 0.4 g per cubic meter of air.

9. The method of claim 1, wherein the malodor in air space, and wherein the malodor counteracting effective amount is from about 4 mg to about 0.2 g per cubic meter of air.

10. A composition for counteracting a malodor in air space or a substrate comprising methyl 3-methyl cyclohexane carboxylate.

11. The composition of claim 10, wherein methyl 3-methyl cyclohexane carboxylate is in an amount of from about 0.005% to about 50% by weight.

12. The composition of claim 10, wherein methyl 3-methyl cyclohexane carboxylate is in an amount of from about 0.01% to about 20% by weight.

13. The composition of claim 10, wherein methyl 3-methyl cyclohexane carboxylate is in an amount of from about 0.05% to about 10% by weight, or is in an amount of from about 0.1% to about 5% by weight.

14. The composition of claim 10, wherein the composition is incorporated in a functional product selected from the group consisting of a room freshener spray, a fragrance diffuser, a candle, a sachet, a clothes deodorant, a detergent, a fabric softener, a fabric refresher, a linen spray, a disposable diaper, a diaper pail deodorant, an antiperspirant, a deodorant, a garbage bag, a car freshener, a pet care product, and an animal litter.

15. Use of methyl 3-methyl cyclohexane carboxylate as a malodor counteracting substance to counteract malodor in air space or in a substrate.
